(19) Europäisches Patentamt
European Patent Office
Office européen des brevets

(11) Veröffentlichungsnummer: **0 070 401**
**A2**

(12) # EUROPÄISCHE PATENTANMELDUNG

(21) Anmeldenummer: **82105498.8**

(22) Anmeldetag: **23.06.82**

(51) Int. Cl.[3]: **C 07 D 493/04**
// (C07D493/04, 307/00, 307/00)

(30) Priorität: **16.07.81 DE 3128102**

(43) Veröffentlichungstag der Anmeldung: **26.01.83**
**Patentblatt 83/4**

(84) Benannte Vertragsstaaten: **CH DE FR GB IT LI SE**

(71) Anmelder: **CASSELLA Aktiengesellschaft, Hanauer Landstrasse 526, D-6000 Frankfurt am Main 61 (DE)**

(72) Erfinder: **Schönafinger, Karl, Dr., Parkstrasse 1, D-8531 Uehlfeld (DE)**

(74) Vertreter: **Urbach, Hans-Georg, Dr. et al, Hanauer Landstrasse 526, D-6000 Frankfurt am Main 61 (DE)**

(54) **Verfahren zur Herstellung von Isosorbid-2-acylaten.**

(57) Bei dem Verfahren zur Herstellung von Isosorbid-2-acylaten wird Isosorbid-2,5-diacylat mit Isosorbid umgesetzt.

EP 0 070 401 A2

0070401

Ref. 3221
Dr.Eu/Ll

Verfahren zur Herstellung von Isosorbid-2-acylaten

Die vorliegende Erfindung betrifft ein Verfahren zur Herstellung von 1,4-3,6-Dianhydro-D-sorbit-2-acylaten, auch Isosorbid-2-acylate genannt, der allgemeinen Formel I

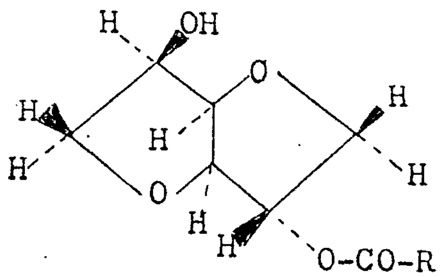

(I)

Die Verbindungen der Formel I sind Acylierungsprodukte des Isosorbids. Diese Verbindung wird z.B. durch Abspaltung von 2 Molen Wasser aus D-Sorbit (engl. Sorbitol) mit Schwefelsäure hergestellt, vgl. Kirk-Othmer, Encyclopedia of Chemical Technology, dritte Auflage, Vol. 1 (1978), Seite 761. Für diese Verbindung ist neben Isosorbid eine Reihe verschiedener Bezeichnungen in Gebrauch, z.B. 1,4:3,6-Dianhydro-D-glucit; 1,4:3,6-Dianhydro-D-glucitol, 1,4-3,6-Dianhydro-D-sorbit; 1,4-3,6-Dianhydrosorbit; Perhydro-furo-/3,2-b/furan-3,6-diol. Entsprechend verschiedene Bezeichnungen werden auch für die Derivate des Isosorbids benutzt.

Aus den erfindungsgemäß herstellbaren 1,4-3,6-Dianhydro-D-sorbit-2-acylaten kann durch Umsetzung mit Salpetersäure und anschließende Abspaltung des Acylrestes das pharmakologisch wirksame 1,4-3,6-Dianhydro-D-sorbit-5-nitrat, auch Isosorbid-5-nitrat genannt, in reiner Form hergestellt werden. Diese Verbindung, auch als 5-ISM (Isosorbid-5-mononitrat) bezeichnet, wird als Heilmittel zur Behandlung von Herzkrankheiten, z.B. Angina pectoris, eingesetzt. Bei der direkten Nitrierung von Isosorbid, nach I.G. Csizmadia und D.L. Hayward, Photochem. Photobiol. 4, 657 (1965) fällt ein Gemisch von Nitraten an, in dem das Isosorbid-5-nitrat nur als untergeordneter Bestandteil anwesend ist. Bei der

BAD ORIGINAL

säulenchromatographischen Isolierung werden deshalb auch nur geringe Ausbeuten an Isosorbid-5-nitrat erhalten.

Bei einem anderen Herstellungsverfahren für Isosorbid-5-nitrat wird zunächst Isosorbid-2,5-dinitrat hergestellt und dieses dann partiell verseift. Aus dem entstehenden Verseifungsgemisch, das Isosorbid-2,5-dinitrat, Isosorbid-5-nitrat, Isosorbid-2-nitrat und Isosorbid enthält, müssen die einzelnen Bestandteile, wie bei dem zuerst genannten Verfahren, durch aufwendige Verfahren voneinander getrennt werden (vgl. Anteunis et al, Org. Magnetic Resonance Vol.3, 363 ff., (1971), D.L. Hayward et al, Can. J. Chem. 45, 2191 ff. (1967)).

Bei einem Verfahren zur Herstellung von 1,4-3,6-Dianhydro-D-glucitolmononitratestern werden die als Ausgangsprodukte benötigten Isosorbid-2- und -5-nitrate durch direkte Nitrierung von Isosorbid hergestellt (vgl. DE-OS 2 221 080). Das Nitrierungsgemisch wird mit Eiswasser behandelt und anschließend mit Ether extrahiert. Der Etherextrakt wird unter Zugabe von Wasser konzentriert und die erhaltene wäßrige Lösung gefriergetrocknet. Durch Kolonnenchromatographie des Nitratgemisches erhält man das Isosorbid-5-nitrat in einer Ausbeute von rund 20 Gew.%.

Bei einem Verfahren zur Herstellung des zu dem Isosorbid-5-nitrat isomeren Isosorbid-2-nitrats wird Isosorbid mit einem Niedrigalkansäureanhydrid oder -chlorid oder -bromid in Gegenwart eines sauren Katalysators acyliert (DE-OS 27 51 934), dann der Isosorbid aus dem Acylierungsgemisch extrahiert, um bei der nachfolgenden Nitrierung die Bildung des explosionsgefährlichen Isosorbid-2,5-dinitrats zu verhindern. In einer dritten Stufe wird das Acylierungsgemisch nitriert und das erhaltene Gemisch aus Isosorbid-5-acylat-2-nitrat, Isosorbid-2-acylat-5-nitrat und Isosorbid-2,5-diacylat mit einer anorganischen Base hydrolysiert. In dem Hydrolysierungsgemisch aus Isosorbid-2-nitrat, Isosorbid-5-nitrat und Isosorbid ist das

Verhältnis Isosorbid-2-nitrat : Isosorbid-5-nitrat größer als 2 : 1, d.h. das Isosorbid-2-nitrat ist in mindestens doppelt so großer Menge vorhanden als das Isosorbid-5-nitrat. Aus dem Hydrolysierungsgemisch wird das Isosorbid-2-nitrat durch Kristallisation isoliert.

Schließlich ist noch ein Verfahren zur Herstellung von Isosorbid-5-nitrat bekannt (DE-OS 29 03 927), das von 1,4-3,6-Dianhydro-mannit, auch Isomannid genannt, ausgeht. Dabei wird Isomannid zunächst mit einem Säurehalogenid einer aromatischen, gegebenenfalls substituierten Benzol- oder Naphthalinsulfonsäure oder einem Säurehalogenid einer Perfluor-$C_{1-4}$-niederalkansulfonsäure, einer $C_{1-4}$-Niederalkansulfonsäure oder einer Perfluor-$C_{1-4}$-niederalkancarbonsäure, einer Carbaminsäure oder der Schwefligsäure umgesetzt. Anstelle der Säurechloride können dabei im Fall der genannten Sulfonsäure, der Perfluor-$C_{1-4}$-niederalkansulfonsäure, der $C_{1-4}$-Niederalkansulfonsäure oder der Perfluor-$C_{1-4}$-niederalkancarbonsäure auch die entsprechenden Anhydride eingesetzt werden. Der erhaltene Isomannid-2-ester wird dann mit einem Alkali- oder Ammoniumsalz einer gegebenenfalls substituierten Benzoesäure oder einer $C_{1-4}$-Niederalkancarbonsäure oder der Ameisensäure umgesetzt, die 5-Hydroxygruppe des erhaltenen Isosorbid-2-esters mit Salpetersäure verestert und das erhaltene Isosorbid-2-ester-5-nitrat selektiv hydrolysiert und/oder umgeestert.

Die vorstehend genannten Herstellungsverfahren für Isosorbid-5-nitrat sind vielstufig, beinhalten umständliche und aufwendige Reinigungsoperationen oder liefern das gewünschte Produkt nur in unbefriedigender Ausbeute oder gehen zudem - wie im Falle des Isomannids - von nicht handelsüblichen Ausgangsprodukten aus.

Es ist auch bereits ein Verfahren zur Herstellung von Isosorbid-5-nitrat vorgeschlagen worden (deutsche Patentanmeldung P 31 17 612.7), bei dem Isosorbid mit einer aliphatischen Carbonsäure zunächst zu einem Acylierungsgemisch

umgesetzt wird, das als Hauptprodukt Isosorbid-2-acylat, als Nebenprodukt Isosorbid-2,5-diacylat und nur Spuren Isosorbid-5-acylat enthält. Das Acylierungsgemisch wird dann ohne oder nach Abtrennung des Isosorbid-2,5-diacylats nitriert und das erhaltene Nitrierungsgemisch zur Abspaltung der Acylgruppen hydrolysiert und/oder umgeestert.

Es wurde nun gefunden, daß 1,4-3,6-Dianhydro-D-sorbit-2-acylate (=Isosorbid-2-acylate) der Formel I einfach und in guten Ausbeuten hergestellt werden können, indem ein 1,4-3,6-Dianhydro-D-sorbit-2,5-diacylat (=Isosorbid-2,5-diacylat) der Formel II

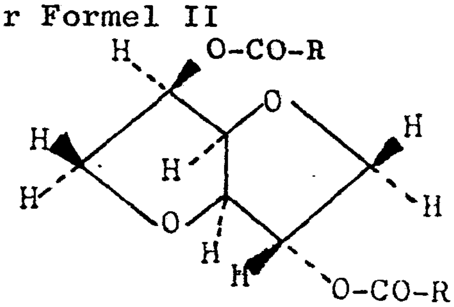

(II)

mit 1,4-3,6-Dianhydro-D-sorbit (=Isosorbid) umgesetzt wird.

In den Formeln I und II bedeutet R Wasserstoff, einen aliphatischen Rest, insbesondere einen Alkylrest mit 1 bis 7 C-Atomen oder Phenyl. Der aliphatische oder Phenyl-Rest kann auch durch Halogen, insbesondere Fluor oder Chlor, $CF_3$, Alkoxy mit 1 bis 4 C-Atomen und der Phenylrest ferner auch durch Alkyl mit 1 bis 4 C-Atomen substituiert sein. Normalerweise sind die aliphatischen Reste und der Phenylrest unsubstituiert. Vorzugsweise bedeutet R

Alkyl mit 1 bis 3 C-Atomen. Die aliphatischen oder Alkyl-Reste können geradkettig oder verzweigt sein. Beispiele für geeignete Rest R sind: n-Butyl, n-Pentyl, n-Hexyl, n-Heptyl, i-Butyl, i-Pentyl, i-Heptyl, 1,1-Dimethyl-ethyl, Vinyl, Alkyl, Phenyl, p-Methyl-phenyl. Bevorzugte Reste R sind: Methyl, Ethyl, Propyl, Isopropyl. Ganz besonders bevorzugt für R ist Methyl.

Die Umsetzung des Isosorbid-2,5-diacylats mit dem Isosorbid wird normalerweise bei Temperaturen von 50 bis 200°C, vorzugsweise 100 bis 170°C, in Gegenwart eines Umesterungskatalysators durchgeführt. Als geeignete Umesterungskatalysatoren kommen alle für diesen Zweck bekannten Verbindungen in Betracht, also z.B. sauer, insbesondere aber basisch, reagierende Verbindungen. Sauer reagierende Umesterungskatalysatoren sind z.B. Mineralsäuren, wie Schwefel-, Salz- oder Phosphorsäure, aromatische oder aliphatische Sulfonsäuren, wie Toluolsulfonsäuren, Methansulfonsäure, Ethansulfonsäure, sauer reagierende Salze, wie Natriumhydrogensulfat sowie in manchen Fällen auch mit Säuren beladene Ionenaustauscher oder Säuregruppen enthaltende polymere Verbindungen. Basisch reagierende Umesterungskatalysatoren sind z.B. Alkalialkoholate, wie Natriummethylat, Kaliumethylat, Kalium-tert.-butylat, Alkalihydroxide, wie Natrium- oder Kaliumhydroxid. Bezogen auf das Gewicht der zur Umsetzung kommenden Ausgangsprodukte wird normalerweise 0,1 bis 15 %, vorzugsweise 0,6 bis 8 %, Katalysator oder Katalysatorgemisch zugesetzt.

Die Umsetzung zwischen dem Isosorbid-2,5-diacylat und dem Isosorbid kann in Anwesenheit eines inerten Lösungsmittels durchgeführt werden. Vorzugsweise wird die Umsetzung jedoch ohne Lösungsmittel durchgeführt.

Bei der Umsetzung zwischen dem Isosorbid-2,5-diacylat und dem Isosorbid kommen bevorzugt äquimolare Mengen zum Einsatz; eine Komponente kann aber auch im Über- oder Unterschuß vorhanden sein. Die Umsetzung ist in der Regel nach 10 Minuten bis 3 Stunden, vorzugsweise 15 Minuten bis 2 Stunden, beendet.

Nach Beendigung der Umsetzung kann das erhaltene Isosorbid-2-acylat leicht, z.B. durch Hochvakuumdestillation und/ oder Umkristallisation abgetrennt und gereinigt werden. Die Ausbeuten an Isosorbid-2-acylat betragen bis zu 90 % der Theorie und mehr.

Die als Ausgangsprodukte benötigten Isosorbid-2,5-diacylate brauchen für die erfindungsgemäße Umsetzung nicht in reiner Form vorzuliegen, sondern können auch wechselnde Mengen der entsprechenden Isosorbid-2-acylate und/oder Isosorbid-5-acylate und/oder des Ausgangsprodukts Isosorbid enthalten. Die Isosorbid-2,5-diacylate können nach verschiedenen Verfahren hergestellt werden, z.B. durch Umsetzung von Isosorbid mit den Carbonsäuren RCOOH in Gegenwart eines sauren Veresterungskatalysators. Solche sauren Katalysatoren sind z.B. Sulfonsäuren, wie z.B. p-Toluolsulfonsäure, Ethansulfonsäure, Methansulfonsäure; nichtoxidierende Mineralsäuren, wie z.B. Schwefelsäure, Salzsäure, Bromwasserstoffsäure, Phosphorsäure; sauer reagierende Salze, wie z.B. Alkalihydrogensulfate, wie z.B. Natriumhydrogensulfat. Anstelle der freien Säuren können auch mit Säuren beladene Ionenaustauscher bzw. Säuregruppen enthaltende polymere Verbindungen eingesetzt werden. In der Regel kommen, bezogen auf Isosorbid, 0,1 bis 15 Gew.%, vorzugsweise 2 bis 6 Gew.%, des sauren Veresterungskatalysators zur Anwendung. Die Herstellung der Isosorbid-2,5-diacylate durch Umsetzung von Isosorbid und der aliphatischen oder aromatischen Carbonsäure RCOOH wird ohne Anwesenheit eines Lösungsmittels, vorzugsweise aber in Anwesenheit eines geeigneten Lösungsmittels, durchgeführt. Bevorzugte Lösungsmittel sind solche, die ein ausreichendes Lösungsvermögen für Isosorbid besitzen und gleichzeitig die Abtrennung des bei der Veresterung des Isosorbids mit der Carbonsäure entstehenden Reaktionswassers durch azeotrope Destillation ermöglichen. Derartige bevorzugte Lösungsmittel sind z.B. Chloroform, Tetrachlorkohlenstoff, Ethylenchlorid, Chlorbenzol, Dimethoxyethan, Diethylenglycoldimethylether, Toluol. Auch Mischungen verschiedener Lösungsmittel können verwendet werden, insbesondere z.B. von Chlorbenzol und Toluol, oder von Ethylenglykoldimethylether und Toluol.

Bei der Herstellung der Isosorbid-2,5-diacylate durch Umsetzung von Isosorbid und Carbonsäuren RCOOH kann das Molverhältnis zwischen Isosorbid und der Carbonsäure in weiten

Grenzen schwanken. Insbesondere wenn die Umsetzung in einem geeigneten Lösungsmittel durchgeführt wird, beträgt das Molverhältnis Isosorbid : Carbonsäure normalerweise 1 : (1,7 bis 2,3), vorzugsweise 1 :(1,8 bis 2,2). Bei der Umsetzung kann jedoch die Carbonsäure auch in einem großen molaren, d.h. z.B. 2- bis 20fachen und noch größeren Überschuß anwesend sein. Bei der Umsetzung des Isosorbids mit der Carbonsäure können pro Mol Isosorbid auch weniger als 1,7 Mole Carbonsäure, z.B. 0,7 bis 1,7 Mole, angewandt werden.Es werden dann Acylierungsgemische erhalten, die neben den Isosorbid-2,5-diacylaten zunehmende Mengen von Isosorbidmono-acylaten, und zwar vorwiegend Isosorbid-2-acylate, enthalten. Derartige Acylierungsgemische können, wie bereits erwähnt, nach dem erfindungsgemäßen Verfahren ohne Reinigung weiter umgesetzt werden, sofern sie noch nicht umgesetztes Isosorbid in ausreichender Menge enthalten, sogar ohne Zusatz von Isosorbid. Derartige Acylierungsgemische des Isosorbids, denen für die weitere Umsetzung nach dem erfindungsgemäßen Verfahren kein oder nur noch wenig Isosorbid zugesetzt werden muß, werden insbesondere bei der sauer katalysierten Acylierung von Isosorbid mit einer Carbonsäure RCOOH im Molverhältnis Isosorbid : RCOOH = 1 : (0,7 bis 1,3), vorzugsweise 1 : (0,8 bis 1,2), erhalten.

Die Umsetzung zwischen Isosorbid und der Carbonsäure wird bei Raumtemperatur oder zweckmäßigerweise bei erhöhter Temperatur, insbesondere bei der Rückflußtemperatur des verwendeten Lösungsmittels, durchgeführt. Dabei findet eine Acylierung oder Veresterung des Isosorbids unter Abspaltung von Wasser statt. Das Fortschreiten der Reaktion kann deshalb durch Bestimmung des abgeschiedenen Wassers verfolgt werden.

Die für das erfindungsgemäße Verfahren als Ausgangsprodukte benötigten Isosorbid-2,5-diacylate oder Acylierungsgemische des Isosorbids, die Isosorbid-2,5-diacylate enthalten, können aber auch, ausgehend von Isosorbid und anderen

Carbonsäurederivaten, wie z.B. Carbonsäurehalogeniden, insbesondere Carbonsäurechloriden, Carbonsäureanhydriden und reaktiven Carbonsäureestern nach zum Teil bekannten Methoden hergestellt werden.

Die nach dem erfindungsgemäßen Verfahren herstellbaren Isosorbid-2-acylate der Formel I, insbesondere diejenigen, bei denen R **Alkyl mit 1 bis 3 C-Atomen** bedeutet, eignen sich hervorragend als Ausgangsprodukte für die Herstellung des pharmakologisch wertvollen Isosorbid-5-nitrats.Hierzu wird das Isosorbid-2-acylat nitriert. Die Nitrierung wird zweckmäßigerweise in einem geeigneten Lösungsmittel, wie z.B. Methylenchlorid, bei Temperaturen von -10 bis 30°C, vorzugsweise von 0 bis 20°C, mit einem aus Acetanhydrid/Eisessig/Salpetersäure oder Schwefelsäure/Salpetersäure oder Essigsäure/Salpetersäure bestehenden Nitriergemisch durchgeführt.

Das bei der Nitrierung erhaltene Produkt besteht hauptsächlich aus Isosorbid-2-acylat-5-nitrat und wird anschließend in an sich bekannter Weise hydrolysiert und/oder umgeestert, wodurch durch Abspaltung der Acylgruppen Isosorbid-5-nitrat entsteht. Auf die genannte Weise kann Isosorbid-5-nitrat, ausgehend von Isosorbid, in einer Gesamtausbeute von über 60 % hergestellt werden.

Beispiel 1

a) 146 g (1 Mol) Isosorbid, 150 g (2,5 Mole) Eisessig und 5 g p-Toluolsulfonsäure werden in 300 ml Toluol am Wasserabscheider erwärmt, bis sich nach ca. 3 bis 4 Stunden kein Wasser mehr abscheidet. Die Reaktionsmischung wird eingeengt, in Wasser aufgenommen und mit Natriumbicarbonat neutral gestellt. Das Isosorbid-2,5-diacetat wird zweimal mit je 300 ml Methylenchlorid ausgeschüttelt und destillativ (Kp 0,67 mbar 125 bis 130°C) gereinigt. Ausbeute: 206 g (89,6 % der Theorie) Isosorbid-2,5-diacetat.

b) 115 g (0,5 Mol) Isosorbid-2,5-diacetat, 73 g (0,5 Mol) Isosorbid und 5 g pulverisiertes Kaliumhydroxid werden eine Stunde lang auf 140°C erhitzt. Das erhaltene Produkt wird bei einem Druck von 0,27 mbar destilliert und das bei 100 bis 105°C übergehende Destillat durch Verrühren in 150 ml Isopropanol bei 0°C zur Kristallisation gebracht. Das Kristallisat wird abgetrennt und getrocknet. Ausbeute: 158 g (88,7 % der Theorie) Isosorbid-2-acetat vom Fp. 78 bis 79°C.

Nach dem vorstehenden Beispiel können z.B. in analoger Weise hergestellt werden: Isosorbid-2-formiat, Isosorbid-2-propionat, Isosorbid-2-butyrat, Isosorbid-2-benzoat.

Beispiel 2

146 g (1 Mol) Isosorbid, 66 g (1,1 Mole) Eisessig und 5 g p-Toluolsulfonsäure werden in 400 ml Toluol ca. 2 bis 3 Stunden am Wasserabscheider erhitzt, bis sich kein Wasser mehr abscheidet. Dann wird im Wasserstrahlvakuum das Toluol abdestilliert und der Rückstand, der Isosorbid-2,5-diacetat, Isosorbid-2-acetat, Isosorbid-5-acetat, und Isosorbid enthält, mit 6 g Kaliumhydroxid-Schuppen versetzt und 1 Stunde lang auf 150°C erhitzt. Dann wird das Produkt bei einem Druck von 0,67 mbar über eine Kolonne destilliert. Bei 108 bis 112°C gehen 147 g (78,2 % der Theorie) Isosorbid-2-acetat über, das sich dünnschichtchromatographisch als fast einheitlich erweist. Durch Umkristallisation aus Isopropanol wird ein dünnschicht-chromatographisch völlig einheitliches Isosorbid-2-acetat vom Fp. 78 bis 79°C erhalten.

Beispiel 3

115 g (0,5 Mol) Isosorbid-2,5-diacetat, 73 g (0,5 Mol) Isosorbid und 5 g p-Toluolsulfonsäure werden eine Stunde lang auf 140°C erwärmt. Danach wird bei 0,27 mbar destilliert, wobei 151 g Isosorbid-2-acetat bei 100 bis 106°C übergehen.

Beispiel 4

Beispiel 2 wurde mit dem Unterschied wiederholt, daß dem

nach dem Abdestillieren des Toluols zurückbleibenden Rückstand kein Kaliumhydroxid zugesetzt wurde. Bei der anschließenden einstündigen Erhitzung des Rückstands auf 150°C wirkte die im Rückstand noch vorhandene p-Toluolsulfonsäure als Umesterungskatalysator. Bei einem Druck von 0,27 mbar wurden bei 100 bis 104°C 157,7 g (83,9 % der Theorie) Isosorbid-2-acetat über eine Vigreux-Kolonne abdestilliert. Das Destillat erwies sich dünnschichtchromatographisch als fast einheitlich. Durch Umkristallisation aus Isopropanol konnte ein dünnschichtchromatographisch einheitliches Isosorbid-2-acetat vom Fp. 78 bis 79°C erhalten werden.

Beispiel 5

5 ml 100 %ige Salpetersäure werden mit einer Spatelspitze Harnstoff versetzt und auf 0°C abgekühlt; 7 ml konzentrierte Schwefelsäure werden zugetropft. Das so erhaltene Nitriergemisch wird in einem Eisbad gut gekühlt. In das gekühlte Nitriergemisch wird die Lösung von 9,5 g Isosorbid-2-acetat in 50 ml Methylenchlorid getropft, eine Stunde bei 0°C nachgerührt und dann auf Eiswasser gegossen. Die Methylenchloridphase wird abgetrennt, getrocknet, mit 100 ml Ethanol versetzt und im Wasserstrahlvakuum auf etwa die Hälfte eingeengt. Nach Zugabe einer Lösung von 1 g Kaliumhydroxid in 20 ml Ethanol wird 5 Minuten lang auf 50°C erwärmt, abgekühlt, mit 25 %iger Salzsäure neutralisiert und eingeengt, nachdem dünnschichtchromatographisch nachgewiesen worden ist, daß kein explosives Isosorbid-2,5-dinitrat vorhanden ist. Nach dem Umkristallisieren aus Toluol erhält man 7,3 g (76 % der Theorie) reines Isosorbid-5-nitrat vom Fp. 90 bis 91°C.

Wenn nach Beispiel 1 hergestelltes Isosorbid-2,5-diacetat eingesetzt wird, beträgt die Ausbeute an Isosorbid-5-nitrat, bezogen auf Isosorbid, 60,4 % der Theorie.

PATENTANSPRÜCHE

1. Verfahren zur Herstellung von 1,4-3,6-Dianhydro-D-sorbit-2-acylaten der Formel I

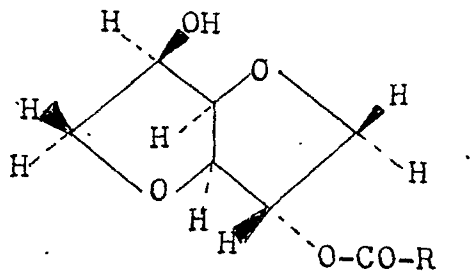

(I)

worin R Wasserstoff oder einen aliphatischen oder aromatischen Rest bedeutet, dadurch gekennzeichnet, daß ein 1,4-3,6-Dianhydro-D-sorbit-2,5-diacylat der Formel II

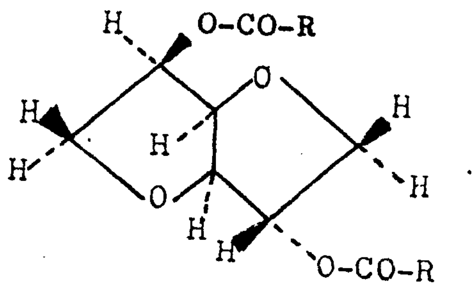

(II)

mit 1,4-3,6-Dianhydro-D-sorbit umgesetzt wird.

2. Verfahren nach Anspruch 1, dadurch gekennzeichnet, daß R Wasserstoff oder einen aliphatischen Rest mit 1 bis 7 C-Atomen oder einen Phenylrest bedeutet, wobei der aliphatische oder Phenylrest durch Halogen, $CF_3$, Alkoxy mit 1 bis 4 C-Atomen und der Phenylrest ferner auch durch Alkyl mit 1 bis 4 C-Atomen substituiert sein kann.

3. Verfahren nach den Ansprüchen 1 und/oder 2, dadurch gekennzeichnet, daß R einen Alkylrest mit 1 bis 3 C-Atomen, vorzugsweise Methyl, bedeutet.

4. Verfahren nach einem oder mehreren der Ansprüche 1 bis 3, dadurch gekennzeichnet, daß die Umsetzung bei Temperaturen von 50 bis 200°C, vorzugsweise 100 bis 170°C, durchgeführt wird.

5. Verfahren nach einem oder mehreren der Ansprüche 1 bis 4, dadurch gekennzeichnet, daß die Umsetzung in Anwesenheit eines Umesterungskatalysators durchgeführt wird.

6. Verfahren nach einem oder mehreren der Ansprüche 1 bis 5, dadurch gekennzeichnet, daß als Ausgangsprodukt der Formel II ein Produkt eingesetzt wird, das durch sauer katalysierte Veresterung von Isosorbid mit einer Carbonsäure RCOOH im Molverhältnis Isosorbid :RCOOH = 1 : (0,7 bis 2,3) erhalten worden ist.

7. Verfahren nach einem oder mehreren der Ansprüche 1 bis 6, dadurch gekennzeichnet, daß als Gemisch eines 1,4-3,6-Dianhydro-D-sorbit-2,5-diacylats der Formel II und 1,4-3,6-Dianhydro-D-sorbit ein Acylierungsgemisch eingesetzt wird, das durch sauer katalysierte Veresterung von 1,4-3,6-Dianhydro-D-sorbit mit einer Carbonsäure RCOOH im Molverhältnis 1 : (0,7 bis 1,3), vorzugsweise 1 :(0,8 bis 1,2), erhalten worden ist.

8. Verwendung der nach einem oder mehreren der Ansprüche 1 bis 7 hergestellten 1,4-3,6-Dianhydro-D-sorbit-2-acylate zur Herstellung von 1,4-3,6-Dianhydro-D-sorbit-5-nitrat, dadurch gekennzeichnet, daß das 1,4-3,6-Dianhydro-D-sorbit-2-acylat, insbesondere ein solches, bei dem in der Formel I R = **Alkyl mit 1 bis 3 C-Atomen ist,**
nitriert und das erhaltene Nitrierungsgemisch zur Abspaltung der Acylgruppe hydrolysiert und/oder umgeestert wird.

9. Verwendung der nach einem oder mehreren der Ansprüche 1 bis 7 hergestellten 1,4-3,6-Dianhydro-D-sorbit-2-acylate gemäß Anspruch 8, dadurch gekennzeichnet, daß die Nitrierung in einem Lösungsmittel bei Temperaturen von 0 bis 20°C durchgeführt wird.

10. Verwendung der nach einem oder mehreren der Ansprüche 1 bis 7 hergestellten 1,4-3,6-Dianhydro-D-sorbit-2-acylate nach Anspruch 8 und/oder 9, dadurch gekennzeichnet, daß

das erhaltene Nitrierungsgemisch zur Abspaltung der Acylgruppen mit einer wäßrigen Alkalihydroxidlösung bei Temperaturen von 0 bis 50°C, vorzugsweise 0 bis 40°C, hydrolysiert oder mit einem Alkohol bei Temperaturen von 10 bis 60°C, vorzugsweise 20 bis 60°C, in Gegenwart einer katalytischen Menge einer Base umgeestert wird.